# EUROPÄISCHE PATENTSCHRIFT

(11) **EP 2 758 094 B1**
(45) Veröffentlichungstag und Bekanntmachung des Hinweises auf die Patenterteilung: **23.03.2016**
(21) Anmeldenummer: 12751453.7
(22) Anmeldetag: 17.08.2012
(51) Int. Cl.: A61M 1/36

(54) **ANSCHLUSSKLEMME FÜR EINEN FEUCHTIGKEITSSENSOR ZUR ÜBERWACHUNG EINES GEFÄSSZUGANGS**
TERMINAL CLAMP FOR A MOISTURE SENSOR FOR MONITORING ACCESS TO A VESSEL
SYSTÈME DE PINCE FORMANT BORNE DE CONNEXION POUR UN CAPTEUR D'HUMIDITÉ SERVANT À SURVEILLER UN ABORD VASCULAIRE

(30) Priorität: 21.09.2011 DE 102011113839; 21.09.2011 US 201161537082 P
(43) Veröffentlichungstag der Anmeldung: 30.07.2014
(73) Patentinhaber: Fresenius Medical Care Deutschland GmbH, 61352 Bad Homburg v.d.H. (DE)
(72) Erfinder: HEPPE, John, 66606 St. Wendel (DE); LIPPERT, Simone, 97737 Gemünden am Main (DE); WÜPPER, Andreas, 64572 Büttelborn (DE)
(74) Vertreter: Oppermann, Frank
(86) Internationale Anmeldenummer: PCT/EP2012/003518
(87) Internationale Veröffentlichungsnummer: WO 2013/041174

(56) Entgegenhaltungen:
- WO-A1-99/26686
- WO-A1-2010/091852
- WO-A1-2010/136837
- WO-A2-2009/075592
- US-A- 4 369 794

## Beschreibung

Die Erfindung betrifft eine Anschlussklemme für einen auf die Haut eines Patienten aufzulegenden Feuchtigkeitssensor zur Überwachung eines Gefäßzugangs zu einem Patienten. Darüber hinaus betrifft die Erfindung eine Anordnung, die den Feuchtigkeitssensor und die Anschlussklemme umfasst. Des weiteren betrifft die Erfindung eine Vorrichtung zur Überwachung eines Gefäßzugangs bei einer extrakorporalen Blutbehandlung, bei der Blut eines Patienten über eine arterielle Schlauchleitung, die eine arterielle Kanüle aufweist, von dem Patienten abgeführt wird, und über eine venöse Schlauchleitung, die eine venöse Kanüle aufweist, dem Patienten zugeführt wird, wobei die Vorrichtung zur Überwachung des Gefäßzugangs über den Feuchtigkeitssensor und die Anschlussklemme verfügt.

Auf dem Gebiet der Medizintechnik sind verschiedene Einrichtungen bekannt, mit denen über eine Schlauchleitung Patienten Flüssigkeiten entnommen oder Flüssigkeiten dem Patienten zugeführt werden können. Dabei erfolgt der Zugang zu den Patienten im Allgemeinen mit einem Katheter zum Einführen in Körperorgane oder einer Kanüle zum Punktieren von Gefäßen. Während der Untersuchung oder Behandlung ist ein ordnungsgemäßer Zugang zu dem Patienten sicherzustellen. Daher ist es erforderlich, den Patientenzugang zu überwachen.

Einen ordnungsgemäßen Zugang zu dem Patienten setzen insbesondere auch die extrakorporalen Blutbehandlungsvorrichtungen voraus, die über einen extrakorporalen Blutkreislauf verfügen. Zu den bekannten extrakorporalen Blutbehandlungsvorrichtungen zählen beispielsweise Dialysevorrichtungen und Zellseparatoren, die ein Zugang zu dem Gefäßsystem des Patienten erforderlich machen. Bei der extrakorporalen Blutbehandlung wird dem Patienten über eine arterielle Schlauchleitung mit einer arteriellen Punktionskanüle Blut entnommen, das dem Patienten über eine venöse Schlauchleitung mit einer venösen Punktionskanüle wieder zugeführt wird.

Aus der WO 2006/008866 A1 und US 6,445,304 B1 sind Feuchtigkeitssensoren bekannt, die auf die Haut des Patienten aufgelegt werden. Die Feuchtigkeitssensoren weisen elektrische Kontaktstellen auf, an denen über ein Verbindungskabel die Auswerteinheit der Vorrichtung zur Überwachung des Gefäßzugangs angeschlossen wird.

An die Anschlussklemmen für die Feuchtigkeitssensoren zur Überwachung des Gefäßzugangs bei einer extrakorporalen Blutbehandlung werden besondere Anforderungen gestellt. Die Anschlussklemmen dürfen sich zum einen von dem Feuchtigkeitssensor während der extrakorporalen Blutbehandlung nicht lösen. Zum anderen muss eine sichere elektrische Kontaktierung gewährleistet sein.

Die WO 99/26686 beschreibt eine elektrische Anschlussklemme für einen auf die Haut des Patienten aufzulegenden Sensor zur Messung des elektrischen Widerstands. Die bekannte Anschlussklemme weist eine untere und eine obere Klemmbacke auf, wobei an der oberen Klemmbacke Anschlusskontakte vorgesehen sind, die bei geschlossener Anschlussklemme die elektrischen Kontaktstellen des Sensors kontaktieren. Die beiden Klemmbacken sind mittels einer Feder in die Schließstellung federnd vorgespannt.

Aus der WO 2010/136837 A1 ist eine elektrische Anschlussklemme für medizinische Sensoren bekannt, die über eine untere und obere Klemmplatte verfügt, die gelenkig miteinander verbunden sind. Die obere Klemmplatte kann einrastend an der unteren Klemmplatte fixiert werden. Der medizinische Sensor wird zwischen den beiden Klemmplatten verklemmt, wobei die Kontaktstellen des Sensors mit den Anschlusskontakten der Klemmplatten kontaktieren. Zur Fixierung der oberen Klemmplatte an der unteren Klemmplatte verfügt die untere Klemmplatte über seitliche Stege, die Bohrungen aufweisen, in die Ansätze einrastend eingreifen, die an den Seiten eines mittleren Abschnitts der oberen Klemmplatte vorgesehen sind. Die beiden seitlichen Stege bilden seitliche Begrenzungen zur Führung der Anschlusslasche des Feuchtigkeitssensors.

Die US 4,369,794 A beschreibt eine konventionelle Schlauchklemme, die eine elektrische Kontaktierung von elektrischen Leitern erlaubt, die in der Schlauchleitung vorgesehen sind. Die Schlauchklemme weist einen oberen und unteren Schenkel auf, die an ihren hinteren Endstücken miteinander verbunden sind. Der untere Schenkel weist an seinem vorderen Endstück zwei im Abstand zueinander angeordnete seitliche Arme auf, an deren Innenseiten sich Ausnehmungen befinden, in die Ansätze des vorderen Endstücks des oberen Schenkels einrastend greifen.

Der Erfindung liegt die Aufgabe zugrunde, eine Anschlussklemme für einen Feuchtigkeitssensor zur Überwachung eines Gefäßzugangs zu schaffen, die sich in großen Stückzahlen kostengünstig herstellen lässt und auch unter mechanischen Belastungen eine sichere elektrische Kontaktierung erlaubt.

Darüber hinaus liegt der Erfindung die Aufgabe zugrunde, eine Anordnung mit einer derartigen Anschlussklemme und einem Feuchtigkeitssensor zur Überwachung eines Gefäßzugangs sowie eine Vorrichtung zur Überwachung eines Gefäßzugangs für eine extrakorporale Blutbehandlungsvorrichtung bereitzustellen, die über einen Feuchtigkeitssensor und eine derartige Anschlussklemme verfügt.

Die Lösung dieser Aufgaben erfolgt erfindungsgemäß mit den Merkmalen der unabhängigen Patentansprüche. Vorteilhafte Ausführungsformen der Erfindung sind Gegenstand der Unteransprüche.

Die erfindungsgemäße Anschlussklemme ist für den Anschluss eines auf die Haut eines Patienten aufzulegenden Feuchtigkeitssensors bestimmt, der über eine Anschlusslasche verfügt.

Die erfindungsgemäße Anschlussklemme weist einen ersten, unteren Schenkel und einen zweiten, oberen Schenkel zur klemmenden Fixierung der Anschlusslasche des Feuchtigkeitssensors auf. Das hintere Endstück des unteren Schenkels und das hintere Endstück des oberen Schenkels sind miteinander verbunden. Zur Kontaktierung der elektrischen Kontaktstellen der Anschlusslasche des Feuchtigkeitssensors verfügt die Anschlussklemme über Anschlusskontakte. In Abhängigkeit von der Ausbildung des Feuchtigkeitssensors können mehr als zwei Anschlusskontakte, beispielsweise vier Anschlusskontakte vorgesehen sein.

Die erfindungsgemäße Anschlussklemme zeichnet sich durch ein Führungsstück aus, das eine schlitzförmige Ausnehmung zur Aufnahme der Anschlusslasche des Feuchtigkeitssensors aufweist. Die schlitzförmige Ausnehmung des Führungsstücks wird von zwei Anschlagelementen seitlich begrenzt, so dass die Anschlusslasche in der Anschlussklemme in einer vorgegebenen Position gehalten wird, in der die Anschlusskontakte der Anschlussklemme mit den elektrischen Kontaktstellen der Anschlusslasche kontaktieren.

Bei der erfindungsgemäßen Anschlussklemme dient die schlitzförmige Ausnehmung des Führungsstücks gleichsam der Arretierung des oberen Schenkels an dem unteren Schenkel. Der obere Schenkel weist Rastmittel auf, die bei geschlossener Anschlussklemme in der schlitzförmigen Ausnehmung einrastend festgelegt sind. Das Führungsstück dient also nicht nur der Führung der Anschlusslasche beim Einschieben des Feuchtigkeitssensors in die Anschlussklemme, sondern auch der Arretierung der beiden Schenkel, wenn die Anschlussklemme geschlossen ist.

Bei einer bevorzugten Ausführungsform weist das vordere Endstück des oberen Schenkels eine dem vorderen Endstück des unteren Schenkels zugewandte Kontaktfläche auf, an der die Anschlusskontakte angeordnet sind. Das vordere Endstück des unteren Schenkels weist vorzugsweise eine dem vorderen Endstück des oberen Schenkels zugewandte Auflagefläche für die Anschlusslasche auf. Es ist grundsätzlich aber auch möglich, dass der untere Schenkel als Kontaktfläche und der obere Schenkel als Auflagefläche ausgebildet ist. Auch können beide Schenkel als Kontaktflächen mit Anschlusskontakten ausgebildet sein, wenn sowohl an der Ober- als auch Unterseite der Lasche des Feuchtigkeitssensors Kontaktstellen vorgesehen sind.

Bei einer weiteren bevorzugten Ausführungsform weist die Auflagefläche für die Anschlusslasche des Feuchtigkeitssensors ein Auflageelement auf, das aus einem elastischen Material besteht. Das nachgiebige Auflageelement stellt eine kraftschlüssige Verbindung zwischen den Anschlusskontakten der Klemme und den elektrischen Kontaktstellen des Feuchtigkeitssensors sicher. Das auf der den Kontaktstellen des Feuchtigkeitssensors gegenüberliegenden Seite angeordnete Auflageelement gleicht beim Zusammendrücken der Schenkel mögliche Toleranzen aus, so dass die Anschlusskontakte des Feuchtigkeitssensors gleichmäßig auf den Kontaktstellen der Anschlusslasche aufliegen.

Eine weitere besonders bevorzugte Ausführungsform sieht vor, dass das vordere Endstück des oberen Schenkels und das vordere Endstück des unteren Schenkels derart ausgebildet sind, dass die Kontaktfläche und die Auflagefläche bei geschlossener Anschlussklemme zueinander parallel ausgerichtet sind. Dadurch wird sichergestellt, dass die Anschlusskontakte parallel zu den Kontaktstellen ausgerichtet sind. Dies kann durch eine geeignete Geometrie der Schenkel der Anschlussklemme erreicht werden. Damit werden Wackelkontakte vermieden und eine sichere Kontaktierung gewährleistet.

Bei einer weiteren besonders bevorzugten Ausführungsform weisen die seitlichen Anschlagelemente des Führungsstücks an den einander gegenüberliegenden Innenseiten jeweils eine Fase auf, mit der die Anschlusslasche beim Einführen in die Anschlussklemme zentriert wird. Dadurch wird das Einführen der Anschlusslasche erleichtert.

Das Führungsstück weist oberhalb der schlitzförmigen Ausnehmung vorzugsweise ein Griffstück zum Öffnen der Anschlussklemme auf. Das Griffstück wirkt dabei als Hebel, mit dem sich das Führungsstück soweit verbiegen lässt, dass das Führungsstück des unteren Schenkels die Rastmittel des oberen Schenkels freigeben.

Die unteren Endstücke des oberen und unteren Schenkels sind vorzugsweise einstückig, so dass sich die Anschlussklemme im Spritzgießverfahren günstig in großen Stückzahlen herstellen lässt. Die einteilige Ausbildung der Anschlussklemme als Spritzgussteil ohne Ritzen oder Nischen hat den Vorteil, dass sich die Schlauchklemme besonders gut reinigen lässt. Beispielsweise kann die Schlauchklemme mit einem Tauchdesinfektions-Verfahren desinfiziert werden. Im Übrigen können sich' Verschmutzungen nur schwer ansammeln, da im Gegensatz zu den bekannten Klemmen, die aus mehreren Elementen, beispielsweise oberen und unteren Teilstücken, Verbindungsstücken, Federn etc. bestehen, Ritzen oder Nischen, in denen sich Verschmutzungen leicht ansammeln könnten, nicht vorhanden sind.

Die hinteren Endstücke des oberen und unteren Schenkels sind vorzugsweise als ein Verbindungsstück ausgebildet, das den oberen und unteren Schenkel in die geöffnete Stellung federnd vorspannt. Dabei sind die oberen und unteren Schenkel und das Verbindungsstück vorzugsweise derart ausgebildet, dass sich beim Öffnen und Schließen der Anschlussklemme der obere und untere Schenkel nur unwesentlich verformt. Da sich die beiden Schenkel nicht verformen, kann eine planparallele Anordnung der Anschlusskontakte der Anschlussklemme und der Anschlussstellen des Feuchtigkeitssensors erzielt werden.

Vorzugsweise sind die hinteren Endstücke des oberen und unteren Schenkels als ein Verbindungsstück mit einem Querschnitt ausgebildet, der bei geöffneter Anschlussklemme im Wesentlichen halbkreisförmig ist. Damit wird bei verhältnismäßig einfacher Geometrie und relativ niedrigen mechanischen Bauteilspannungen eine handliche Anschlussklemme geschaffen, die verhältnismäßig geringe Abmessungen hat.

Die Anschlussklemme weist vorzugsweise eine Buchse zum Anschluss eines Steckers eines Verbindungskabels auf. An der Anschlussklemme kann aber auch ein Stecker vorgesehen sein, der sich an eine Buchse anschließen lässt. Das Verbindungskabel kann aber auch unlösbar mit der Anschlussklemme verbunden oder aus der Anschlussklemme herausgeführt sein.

Um die Anschlussklemme beim Zusammendrücken der Schenkel besser greifen zu können, weist einer der beiden Schenkel, vorzugsweise der untere Schenkel der Anschlussklemme eine Griffmulde auf. Die Griffmulde erlaubt es dem Anwender die Anschlussklemme leicht zu greifen und beim Öffnen bzw. Schließen der Klemme eine gezielte Gegenkraft auszuüben. Dadurch wird verhindert, dass die Anschlussklemme beim Öffnen bzw. Schließen auf den Arm des Patienten gedrückt wird.

Die Anschlusskontakte der Anschlussklemme können unterschiedlich ausgebildet sein. Die Kontakte können eine ballige Kontaktfläche aufweisen. Sie können auch federnd vorgespannte Kontaktelemente aufweisen. Dadurch werden unabhängig von der Klemmkraft der Anschlussklemme die Kontaktelemente der Anschlusskontakte mit ausreichender Anpresskraft auf die Kontaktstellen der Anschlusslasche gedrückt. Dabei werden auch mögliche Toleranzen, z.B. Bauteiltoleranzen, beispielsweise Geometrietoleranzen, Längentoleranzen, ausgeglichen. Anschlusskontakte mit federnden Kontaktpins sind dem Fachmann allgemein bekannt.

Im Folgenden wird ein Ausführungsbeispiel der Erfindung unter Bezugnahme auf die Zeichnungen näher erläutert.

Es zeigen:
- Fig. 1: die wesentlichen Komponenten einer extrakorporalen Blutbehandlungsvorrichtung, die über eine Vorrichtung zur Überwachung des Gefäßzugangs verfügt.
- Fig. 2: eine Seitenansicht der geschlossenen Anschlussklemme zum Anschluss des Feuchtigkeitssensors der Vorrichtung zur Überwachung des Gefäßzugangs,

- Fig. 3: die geöffnete Anschlussklemme,
- Fig. 4: die Anschlussklemme in der Draufsicht,
- Fig. 5: ein Anschlusskontakt der Anschlussklemme,
- Fig. 6: die Seitenansicht der Anschlussklemme in perspektivischer Darstellung,
- Fig. 7: eine perspektivische Darstellung der Anschlussklemme in der Rückansicht,
- Fig. 8: die Anschlussklemme mit einer ersten Ausführungsform des Feuchtigkeitssensors
- Fig. 9: die Anschlussklemme mit einer zweiten Ausführungsform des Feuchtigkeitssensors
- Fig. 10: die Anschlusslasche einer weiteren Ausführungsform des Feuchtigkeitssensors,
- Fig. 11: die Anschlussklemme für den Feuchtigkeitssensor von Fig. 10 in der Seiten- und Vorderansicht und
- Fig. 12: die in die Anschlussklemme von Fig. 11 eingesetzte Anschlusslasche des Feuchtigkeitssensors von Fig.10.

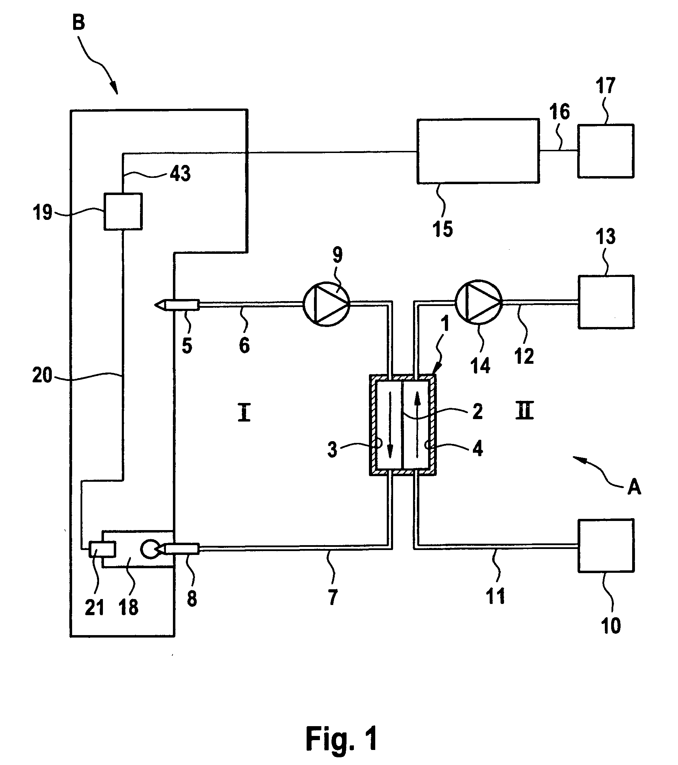

Fig. 1 zeigt die wesentlichen Komponenten einer extrakorporalen Blutbehandlungsvorrichtung A, insbesondere Hämodialysevorrichtung, die über eine Vorrichtung B zur Überwachung des Gefäßzugangs verfügt. Bei dem vorliegenden Ausführungsbeispiel ist die Überwachungsvorrichtung B Bestandteil der Hämodialysevorrichtung A.

Die Hämodialysevorrichtung A weist einen Dialysator 1 auf, der durch eine semipermeable Membran 2 in eine Blutkammer 3 und eine Dialysierflüssigkeitskammer 4 unterteilt ist. An der Fistel oder dem Shunt des Patienten ist mittels einer arteriellen Punktionskanüle 5 eine arterielle Schlauchleitung 6 angeschlossen, die zu einem Einlass der Blutkammer 3 des Dialysators 1 führt. Von dem Auslass der Blutkammer 3 des Dialysators 1 geht eine venöse Schlauchleitung 7 ab, die mittels einer venösen Punktionskanüle 8 an der Fistel oder dem Shunt des Patienten angeschlossen ist. In die arterielle Schlauchleitung 6 ist eine Blutpumpe 9 geschaltet, die das Blut im extrakorporalen Blutkreislauf I fördert.

Der Dialysierflüssigkeitskreislauf II der Dialysevorrichtung A umfasst eine Dialysierflüssigkeitsquelle 10, an der eine Dialysierflüssigkeitszuführleitung 11 angeschlossen ist, die zu dem Einlass der Dialysierflüssigkeitskammer 4 des Dialysators führt. Von dem Auslass der Dialysierflüssigkeitskammer 4 des Dialysators 1 geht eine Dialysierflüssigkeitsabführleitung 12 ab, die zu einem Auslass 13 führt. In die Dialysierflüssigkeitsabführleitung 12 ist eine Dialysierflüssigkeitspumpe 14 geschaltet.

Die Steuerung der Dialysevorrichtung erfolgt mit einer zentralen Steuereinheit 15, die über eine Datenleitung 16 mit einer Alarmeinheit 17 verbunden ist.

Die Überwachungsvorrichtung B verfügt über einen Feuchtigkeitssensor 18, der auf die Haut des Patienten aufgelegt wird. Der Feuchtigkeitssensor 18 ist in Fig. 1 nur schematisch dargestellt. Darüber hinaus verfügt die Überwachungsvorrichtung B über eine Auswerteinheit 19, die über ein Verbindungskabel 20 an den Feuchtigkeitssensor 18 angeschlossen ist. Zum Anschluss des Feuchtigkeitssensors 18 dient eine nur schematisch dargestellte Anschlussklemme 21, die nachfolgend noch im Einzelnen beschrieben wird.

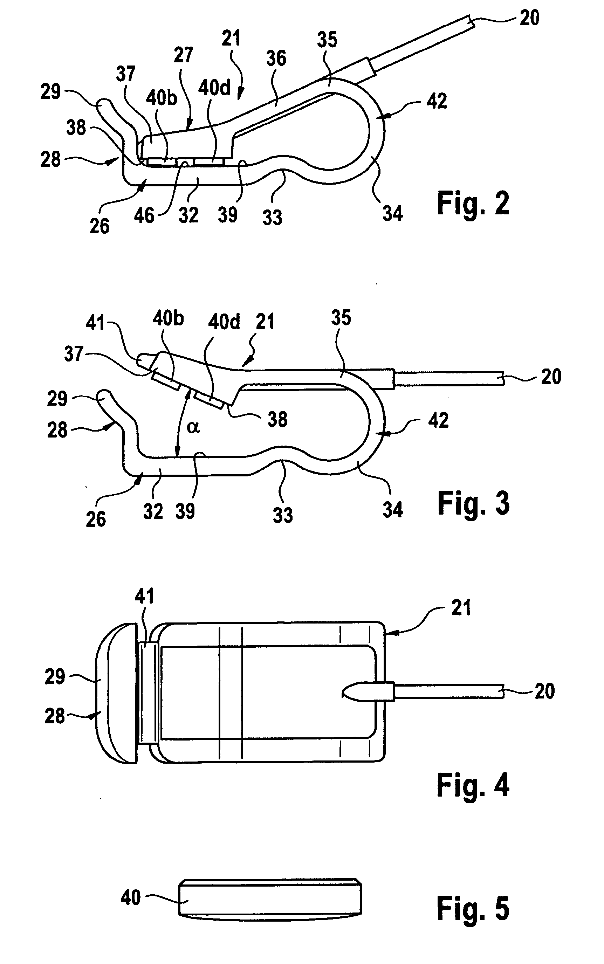

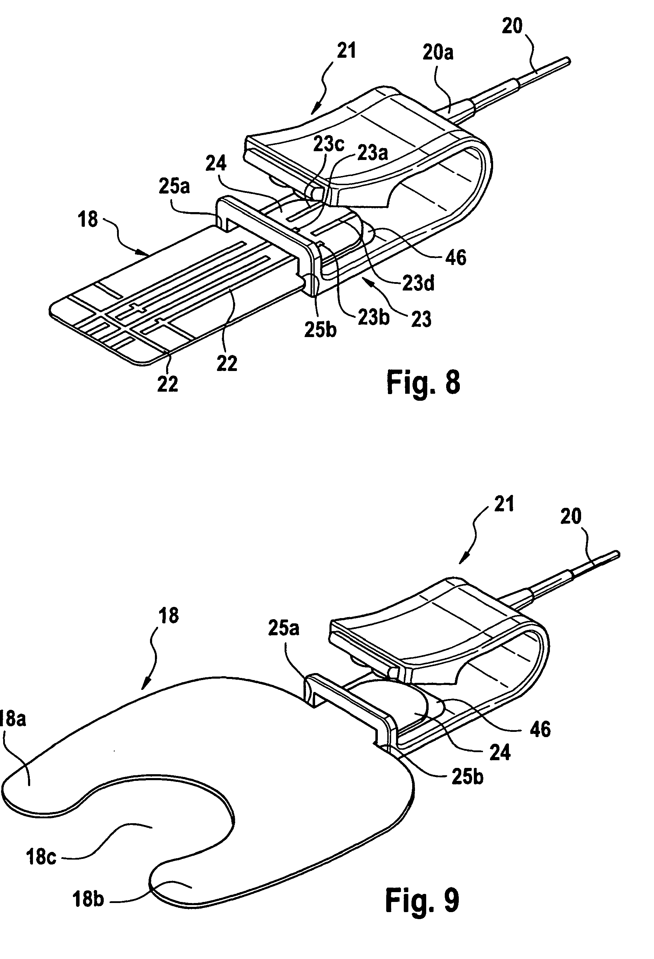

Die Fig. 8 und 9 zeigen die Anschlussklemme 21 zusammen mit dem Feuchtigkeitssensor. Fig. 8 zeigt ein erstes Ausführungsbeispiel des Feuchtigkeitssensors 18, während Fig. 2 ein zweites Ausführungsbeispiel des Feuchtigkeitssensors 18 zeigt.

Der Feuchtigkeitssensor 18 weist eine Anordnung von in Fig. 8 nur andeutungsweise dargestellten Leiterbahnen 22 auf, die eine Widerstandsmessung erlauben. Die Leiterbahnen 22 führen zu Kontaktstellen 23, um einen elektrischen Kontakt mit entsprechenden Anschlusskontakten der Anschlussklemme 21 herstellen zu können. Die Anschlusskontakte 23 sind auf einer Anschlusslasche 24 des Feuchtigkeitssensors 18 angeordnet.

Fig. 8 zeigt einen Feuchtigkeitssensor 18 mit einem rechteckförmigen Pad, das an die Anschlusslasche 24 angeformt ist, während Fig. 9 einen Feuchtigkeitssensor 18 mit einem Pad zeigt, das zwei Schenkel 18a und 18b aufweist, die eine halbkreisförmige Ausnehmung 18c umschließen.

Die Anschlusslaschen 24 beider Feuchtigkeitssensoren 18 haben die gleichen Abmessungen. Auf der Oberseite der Anschlusslasche 24 befinden sich bei den vorliegenden Ausführungsbeispielen zwei vordere Kontaktstellen 23a und 23b sowie zwei hintere Kontaktstellen 23c und 23d. Der Übergang zwischen der Anschlusslasche und dem Pad mit den Leiterbahnen 22 bildet einen Ansatz 25a und 25b, mit dem der Feuchtigkeitssensor 18 an der Anschlussklemme 21 anliegt, wenn die Anschlusslasche in der Anschlussklemme liegt.

Nachfolgend wird die Anschlussklemme 21 für den Feuchtigkeitssensor 18 im Einzelnen beschrieben.

Fig. 2 zeigt die Anschlussklemme 21 in der geschlossenen und Fig. 3 in der geöffneten Stellung. Die Anschlussklemme 21 ist in der Art eines Clip ausgebildet, der als einstückiges Spritzgießteil aus Kunststoff hergestellt ist. Sie weist einen der Haut des Patienten zugewandten unteren Schenkel 26 und einen oberen Schenkel 27 auf, die einstückig sind. Beide Schenkel 26, 27 sind flache Kunststoffteile.

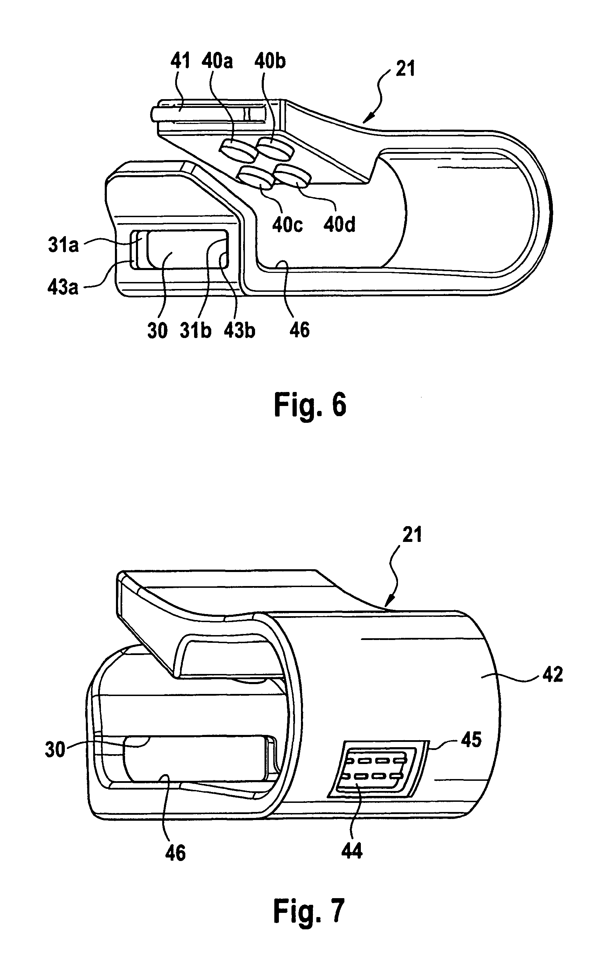

Das vordere Endstück des unteren Schenkels 26 ist als Führungsstück 28 zur Aufnahme der Anschlusslasche 24 des Feuchtigkeitssensors 18 ausgebildet. Das Führungsstück 28 weist eine schlitzförmige Ausnehmung 30 auf, die sich über die Breite der Anschlussklemme 21 erstreckt (Fig. 6). Die seitlichen Begrenzungen der schlitzförmigen Ausnehmung 30 bilden dabei Anschlagelemente 31a und 31b für die Ansätze 25a und 25b des Feuchtigkeitssensors 18.

An das obere Ende des Führungsstücks 28 ist ein Griffstück 29 angeformt, das sich schräg nach oben erstreckt. Der untere Schenkel 26 weist ein gerades mittleres Schenkelstück 32 auf, das sich an das Führungsstück 28 anschließt. In dem mittleren Schenkelstück 32 ist eine Griffmulde 33 ausgebildet. Das mittlere Schenkelstück 32 ist am hinteren Endstück 34 nach oben gebogen. An das hintere Endstück 34 des unteren Schenkels 26 schließt sich das nach unten gebogene hintere Endstück 35 des oberen Schenkels 27 an, das in ein gerades mittleres Schenkelstück 36 übergeht. Das vordere Endstück 37 des oberen Schenkels 27 ist gegenüber dem mittleren Schenkelstück 36 bei geöffneter Anschlussklemme 21 nach oben schräg gestellt.

Das vordere Endstück 37 des oberen Schenkels 27 weist im geschlossenen Zustand der Anschlussklemme 21 eine dem unteren Schenkel 26 zugewandte plane Kontaktfläche 38 auf, während das mittlere Schenkelstück 32 des unteren Schenkels 26 eine der Kontaktfläche 38 des oberen Schenkels 27 zugewandte plane Auflagefläche 39 aufweist. Die Kontaktfläche 38 und Auflagefläche 39 schließen im geöffneten Zustand der Anschlussklemme 21 einen Winkel α ein, der bei dem vorliegenden Ausführungsbeispiel 25° beträgt.

An der Kontaktfläche 38 sind die Anschlusskontakte 40 der Anschlussklemme 21 angeordnet. Bei dem vorliegenden Ausführungsbeispiel weist die Anschlussklemme 21 zwei vordere und zwei hintere Anschlusskontakte auf, die im gleichen Abstand zueinander wie die Kontaktstellen 23a - 23d des Feuchtigkeitssensors 18 angeordnet sind. Die Anschlusskontakte sind in einer vorderen und einer hinteren Reihe angeordnet, wobei die beiden Anschlusskontakte 40a, 40b der vorderen Reihe zu den beiden Schlusskontakten 40c, 40d der hinteren Reihe versetzt angeordnet sind. Die vier Anschlusskontakte 40a bis 40d liegen somit auf den Ecken einer gedachten Raute. Der Vorteil dieser Anordnung liegt darin, dass die Kontaktflächen der Anschlusskontakte bei gegebenem Platzangebot auf der Klemme möglichst groß ausgebildet werden können. Beispielsweise kann der Durchmesser der Kontaktflächen jeweils 4 mm betragen, wobei der Abstand zwischen den Kontakten einer Reihe rund 2,5 mm betragen kann. Wenn die Kontakte alle nebeneinander in einer Reihe lägen, so müsste die Klemme wesentlich breiter ausgeführt werden. Wenn die beiden Reihen nicht versetzt angeordnet wären, so müsste die Klemme länger ausgeführt werden. Demgegenüber ist die erfindungsgemäße Klemme besonders kompakt und kostengünstig.

Fig. 5 zeigt eines der Anschlusskontakte 40 in der Seitenansicht. Die Anschlusskontakte weisen an der Unterseite eine ballige Kontaktfläche auf, die eine sichere Kontaktierung erlaubt. Anstelle der in Fig. 5 gezeigten Anschlusskontakte können aber auch federnd vorgespannte Anschlusspins vorgesehen sein. Derartige Anschlusspins sind dem Fachmann als "gefederte Kontaktstifte" bekannt.

Die Kontaktfläche 38 und die Auflagefläche 39 sind in der geöffneten Stellung der Anschlussklemme 21 (Fig. 3), in der die Anschlussklemme 21 mechanisch entspannt ist, derart ausgerichtet, dass bei geschlossener, also gespannter Anschlussklemme 21 (Fig. 2) die Kontaktfläche 38 parallel zu der Auflagefläche 39 angeordnet ist bzw. die Anschlusskontakte 40a - 40d parallel zu der Auflagefläche 39 angeordnet sind. An die Anschlusskontakte 40a - 40d sind die elektrischen Leitungen des Verbindungskabels 20 angeschlossen, das aus dem hinteren Endstück 35 der Anschlussklemme 21 herausgeführt ist.

Das vordere Endstück 37 des oberen Schenkels 27 weist Rastmittel 41 auf, die in der geschlossenen Stellung der Anschlussklemme 21 (Fig. 2) in die schlitzförmige Ausnehmung 30 einrastend eingreifen, wenn der obere Schenkel 27 und der untere Schenkel 26 zusammengedrückt werden, so dass der obere Schenkel nicht in die geöffnete Stellung (Fig. 3) zurückschnellen kann. Die Rastmittel sind eine sich über die Breite der schlitzförmigen Ausnehmung 30 erstreckende Rastnase 41.

An der Auflagefläche 39 des unteren Schenkels 26 der Anschlussklemme 21 ist zum Toleranzausgleich ein nachgiebiges Auflageelement 46 aus einem elastischen Material vorgesehen, auf dem die Anschlusslasche 24 des Feuchtigkeitssensors 18 aufliegt.

Die Anschlussklemme 21 ist derart ausgebildet, dass das Verbindungsstück 42 des unteren und oberen Schenkels 26, 27 einen im Wesentlichen halbkreisförmigen Querschnitt hat. Das Verbindungsstück 42 wirkt als Biegefeder für den unteren und oberen Schenkel 26, 27, wobei sich das Verbindungsstück 42 im Gegensatz zu den Schenkeln 26, 27 beim Öffnen oder Schließen der Klemme leicht verformt. Für die gewünschte planparallele Anpressung der Anschlusskontakte bei geschlossener Anschlussklemme wäre eine Verformung der Schenkel 26, 27 nachteilig. Um beim Öffnen oder Schließen der Klemme die Verformung stets in den Bereich des Verbindungsstücks 42 zu verlagern, kann das Verbindungsstück in seinem Querschnitt zumindest über einen Teil seiner Länge geschwächt sein.

Der Radius des Verbindungsstücks 42 bleibt beim Schließen der Anschlussklemme nahezu unverändert. Das Verbindungsstück 42 übt bereits bei geschlossener Klemme eine Federkraft auf die beiden Schenkel 26, 27 aus, die aber von den Rastmitteln 41 aufgenommen wird. Beim Lösen der Rastmittel 41 führt die Federkraft des Verbindungsstücks 42 zum Öffnen der Anschlussklemme, bis das Verbindungsstück mechanisch entspannt ist. Ein Handhabungsvorteil ergibt sich dadurch, dass sich die Anschlussklemme beim Abrüsten, beispielsweise nach Beendigung der Dialysebehandlung, nach dem Lösen der Rastmittel selbsttätig öffnet und die Klemme somit besonders leicht von der Lasche des Sensors entfernt werden kann.

Zum Anschluss des Feuchtigkeitssensors 18 an die Auswerteinheit der Überwachungsvorrichtung B wird die Anschlusslasche 24 des Feuchtigkeitssensors 18 in die schlitzförmige Ausnehmung 30 des Führungsstücks 28 der Anschlussklemme 21 soweit eingeschoben, bis die Anschlagelemente 31a und 31b des Führungsstücks 28 an den Ansätzen 25a, 25b des Feuchtigkeitssensors 18 anschlagen. Dann werden beide Schenkel 26, 27 der Anschlussklemme 21 zusammengedrückt, bis die Rastmittel 41 in die schlitzförmige Ausnehmung 30 einrasten (Fig. 2).

Die seitlichen Anschlagelemente 31 a, 31 b des Führungsstücks 28 weisen an den einander gegenüberliegenden Seiten jeweils eine Fase 43a, 43b (Fig. 6) auf, die der Zentrierung der Anschlusslasche 24 des Feuchtigkeitssensors 18 dient, wenn die Anschlusslasche in die schlitzförmige Ausnehmung des Führungsstücks 28 geschoben wird. Die Breite der Anschlusslasche 24 und die Breite der schlitzförmigen Ausnehmung 30 sind derart bemessen, dass die Anschlusslasche 24 genau in das Führungsstück 28 passt.

Das Verbindungskabel 20 kann mit der Anschlussklemme 21 fest verbunden sein (Fig. 2 - 4). Es ist aber auch möglich, dass das Verbindungskabel an einem Ende über einen Stecker 20a verfügt, der in eine passende Buchse 44, die an der Anschlussklemme 21 vorgesehen ist, gesteckt wird (Fig. 7).

Fig. 7 zeigt ein Ausführungsbeispiel der Anschlussklemme 21, bei dem die Buchse 44 in einer Ausnehmung 45 an dem Verbindungsstück 42 der beiden Schenkel 26, 27 angeordnet ist. Die Figuren 8 und 9 zeigen ein Ausführungsbeispiel, bei dem das Kabel unlösbar mit der Klemme verbunden ist, d. h. eine Buchse nicht vorgesehen ist.

Der Stecker 20a und die Buchse 44 können derart konfiguriert sein, dass sich der Stecker 20a bei Überschreiten einer vorbestimmten Zugkraft an dem Verbindungskabel 20 aus der Buchse 44 löst, so dass Stecker 20a und Buchse 44 wie eine Sollbruchstelle wirken. Dies hat den Vorteil, dass unzulässige Zugkräfte am Verbindungskabel 20 nicht über die Anschlussklemme und den Feuchtigkeitssensor auf den Gefäßzugang und/oder die Haut des Patienten am Gefäßzugang übertragen werden können. Ein Herausziehen des Steckers 20a aus der Buchse 44 wird durch die Auswerteinheit 19 erkannt.

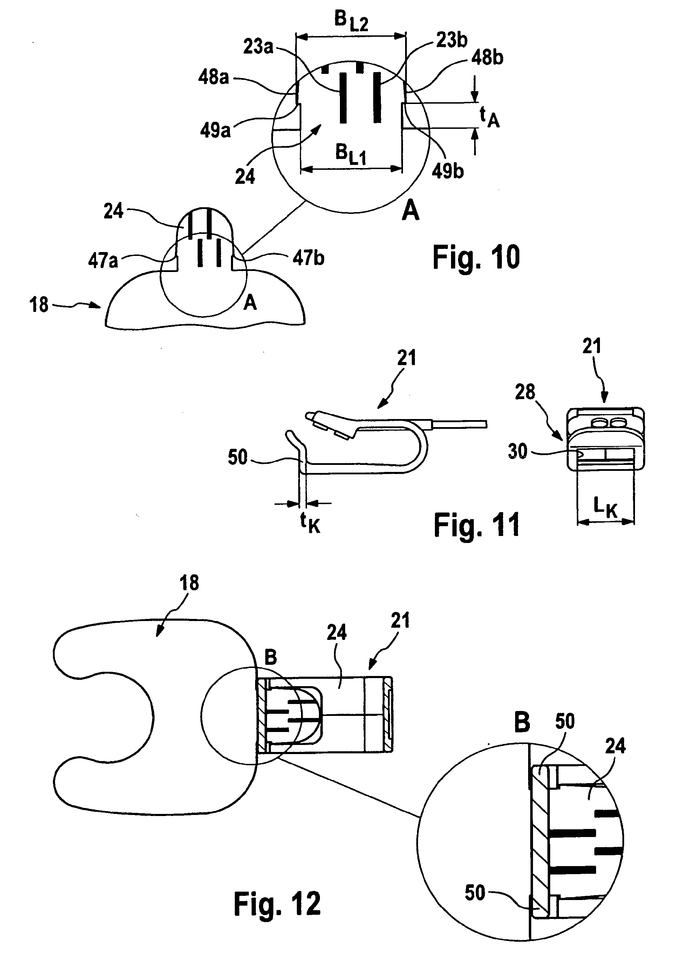

Beim Schließen der Anschlussklemme muss sichergestellt sein, dass die Kontaktstellen 23a bis 23d des Feuchtigkeitssensors 18 exakt auf die Anschlusskontakte 40a bis 40d der Anschlussklemme 21 ausgerichtet sind. Fig. 10 zeigt die Anschlusslasche 24 einer alternativen Ausführungsform des Feuchtigkeitssensors 18, die sich von dem unter Bezugnahme auf Fig. 9 beschriebenen Ausführungsbeispiel dadurch unterscheidet, dass die Lasche 24 einrastend in die schlitzförmige Ausnehmung 30 der Anschlussklemme 21 eingeschoben werden kann, wobei die Lasche gegen Verdrehen in der Anschlussklemme gesichert ist. Die einander entsprechenden Teile sind mit den gleichen Bezugszeichen versehen.

Die Lasche 24 des Feuchtigkeitssensors 18 weist an beiden Längsseiten einen Rasthaken 47a, 47b mit einer vorderen Schrägfläche 48a, 48b und einem hinteren Absatz 49a, 49b auf. In Fig. 10 ist die Breite der Lasche 24 an der schmalsten Stelle mit B_{L1} und an der breitesten Stelle mit B_{L2} bezeichnet, während der Abstand zwischen dem Absatz 49a, 49b und dem Anschlag 25a, 25b des Feuchtigkeitssensors 18 mit t_{A} bezeichnet ist. Die Breite der schlitzförmigen Ausnehmung 30 des Führungsstücks 28 der Anschlussklemme 21 ist in Fig. 11 mit L_{K} bezeichnet. Die Klemme 21 weist an dem Führungsstück 28 einen Anschag 50 mit der Breite t_{K} auf. Für das korrekte Einrasten der Lasche in der Klemme muss B_{L1} < L_{K} < B_{L2} und t_{A} > t_{K} gelten, wobei die maßliche Differenz zwischen B_{L2} und L_{K} fertigungstechnisch sehr klein gehalten werden kann. Die Differenz zwischen B_{L2} und L_{K} ist stark von der Elastizität des Materials der Lasche abhängig, wobei die Lasche vorzugsweise aus einem textilen Gewebe besteht, das sich beim Einführen der Lasche 24 in die Ausnehmung 30 der Klemme 21 elastisch verformt. Zunächst steigt die Kraft zum Verformen der Lasche 24 an der Schrägfläche 48a, 48b linear an, um dann an dem Absatz 49a, 49b schlagartig wieder abzufallen, was als Einrasten deutlich spürbar ist.

Nach dem Einsetzen der Lasche 24 ist der Feuchtigkeitssensor 18 auch gegen Verdrehen gesichert, da beim Verdrehen der Absatz 49a, 49b der Lasche 24 an dem Anschlag 50 der Anschlussklemme 21 anschlägt. Fig. 12 zeigt, dass die Lasche bei korrekter Ausrichtung von Kontaktstellen und Anschlusskontakten aufgrund des Formschlusses verdrehsicher in der Anschlussklemme fixiert ist. Dabei kann ein maximal möglicher Verdrehwinkel mit der Bedingung t_{A}/t_{K} > 1 und B_{L1}/L_{K} < 1 eingestellt werden, wobei der Quotient vorzugsweise so gewählt wird, das ein Einrasten spürbar bleibt.

Die besondere Ausführungsform des Feuchtigkeitssensors hat den Vorteil, dass sich der Sensor auch bei geöffneter Anschlussklemme nicht unbeabsichtigt lösen kann. Dies ist von Vorteil, wenn Sensor und Klemme vor der Behandlung zusammengesteckt werden oder wenn die geöffnete Klemme und der Feuchtigkeitssensor vor Behandlungsbeginn kurz losgelassen werden, um beispielsweise eine Nadelfixierung vornehmen oder eine Gaze einsetzen zu können.

## Patentansprüche

1. Anschlussklemme für einen auf die Haut eines Patienten aufzulegenden Feuchtigkeitssensor zur Überwachung eines Gefäßzugangs, der eine Anschlusslasche aufweist, an der elektrische Kontaktstellen angeordnet sind, mit
einem ersten, unteren Schenkel (26) und einem zweiten, oberen Schenkel (27) zur klemmenden Fixierung der Anschlusslasche, wobei ein hinteres Endstück (34) des unteren Schenkels (26) und ein hinteres Endstück (35) des oberen Schenkels (37) miteinander verbunden sind, und
Anschlusskontakten (40) zur Kontaktierung der elektrischen Kontaktstellen der Anschlusslasche des Feuchtigkeitssensors,
**dadurch gekennzeichnet, dass**
ein vorderes Endstück (37) des oberen Schenkels (27) Rastmittel (41) aufweist und ein vorderes Endstück (28) des unteren Schenkels (26) als Führungsstück mit einer sich über die Breite der Anschlussklemme erstreckenden, schlitzförmigen Ausnehmung (30) zur Aufnahme der Anschlusslasche des Feuchtigkeitssensors ausgebildet ist, die von zwei Anschlagelementen (31a, 31b) seitlich begrenzt ist, wobei die Rastmittel (41) und das Führungsstück (28) derart ausgebildet sind, dass bei geschlossener Anschlussklemme die Rastmittel (41) in der schlitzförmigen Ausnehmung (30) einrastend festgelegt sind.

2. Anschlussklemme nach Anspruch 1, **dadurch gekennzeichnet, dass** das vordere Endstück (37) des oberen Schenkels (27) eine dem vorderen Endstück (28) des unteren Schenkels (26) zugewandte Kontaktfläche (38) aufweist, an der die Anschlusskontakte (40) angeordnet sind.

3. Anschlussklemme nach Anspruch 1 oder 2, **dadurch gekennzeichnet, dass** das vordere Endstück (28) des unteren Schenkels (26) eine dem vorderen Endstück (37) des oberen Schenkels (27) zugewandte Auflagefläche (39) für die Anschlusslasche des Feuchtigkeitssensors aufweist.

4. Anschlussklemme nach Anspruch 3, **dadurch gekennzeichnet, dass** die Auflagefläche (39) für die Anschlusslasche des Feuchtigkeitssensors ein Auflageelement (46) aus einem elastischen Material aufweist.

5. Anschlussklemme nach Anspruch 3 oder 4, **dadurch gekennzeichnet, dass** das vordere Endstück (37) des oberen Schenkels (27) und vordere Endstück (28) des unteren Schenkels (26) derart ausgebildet sind, dass die Kontaktfläche (38) und die Auflagefläche (39) bei geschlossener Anschlussklemme zueinander parallel ausgerichtet sind.

6. Anschlussklemme nach einem der Ansprüche 1 bis 5, **dadurch gekennzeichnet, dass** das Führungsstück (28) oberhalb der schlitzförmigen Ausnehmung (30) ein Griffstück (29) zum Öffnen der Anschlussklemme aufweist.

7. Anschlussklemme nach einem der Ansprüche 1 bis 6, **dadurch gekennzeichnet, dass** die hinteren Endstücke (34, 35) des oberen und unteren Schenkels (26, 27) einstückig sind.

8. Anschlussklemme nach einem der Ansprüche 1 bis 7, **dadurch gekennzeichnet, dass** die hinteren Endstücke (34, 35) des oberen und unteren Schenkels (26, 27) als ein Verbindungsstück (42) ausgebildet sind, dass den oberen und unteren Schenkel (26, 27) in die geöffnete Stellung federnd vorspannt.

9. Anschlussklemme nach Anspruch 8, **dadurch gekennzeichnet, dass** die oberen und unteren Schenkel (26, 27) und das Verbindungsstück (42) derart ausgebildet sind, dass sich beim Öffnen und Schließen der Anschlussklemme der obere und untere Schenkel nicht oder nur unwesentlich verformt.

10. Anschlussklemme nach Anspruch 8 oder 9, **dadurch gekennzeichnet, dass** das Verbindungsstück (42) einen Querschnitt aufweist, der im Wesentlichen halbkreisförmig ist.

11. Anschlussklemme nach einem der Ansprüche 1 bis 10, **dadurch gekennzeichnet, dass** der obere und untere Schenkel (26, 27) der Anschlussklemme als flache Teile aus Kunststoff ausgebildet sind.

12. Anschlussklemme nach einem der Ansprüche 1 bis 11, **dadurch gekennzeichnet, dass** die Anschlussklemme eine Buchse (44) zum Anschluss eines Steckers eines Verbindungskabels aufweist.

13. Anordnung mit einem auf die Haut des Patienten aufzulegenden Feuchtigkeitssensor (18), der eine Anschlusslasche (24) aufweist, an der elektrische Kontaktstellen (23) angeordnet sind, und einer Anschlussklemme (21) nach einem der Ansprüche 1 bis 12, **dadurch gekennzeichnet, dass** die Breite der Anschlusslasche (24) der schlitzförmigen Ausnehmung (30) des Führungsstücks (28) derart bemessen ist, dass die Anschlusslasche passend in das Führungsstück einschiebbar ist.

14. Anordnung nach Anspruch 13, **dadurch gekennzeichnet, dass** die Anschlusslasche (24) an beiden Seiten einen Ansatz (25a, 25b) aufweist, der an den Anschlagelementen (31a, 31b) des Führungsstücks (28) anliegt, wenn die Anschlusslasche (24) in das Führungsstück (28) eingeschoben ist.

15. Vorrichtung zur Überwachung eines Gefäßzugangs für eine extrakorporale Blutbehandlungsvorrichtung, bei der Blut eines Patienten über eine arterielle Schlauchleitung (6), die eine arterielle Kanüle (5) aufweist, von dem Patienten abgeführt wird, und über eine venöse Schlauchleitung (7), die eine venöse Kanüle aufweist (8), dem Patienten zugeführt wird,
mit einem auf die Haut des Patienten aufzulegenden Feuchtigkeitssensor (18), der eine Anschlusslasche (24) aufweist, an der elektrische Kontaktstellen (23) angeordnet sind, und
einer Auswerteinheit (19), die über ein Verbindungskabel (20) an den Feuchtigkeitssensor (18) anschließbar ist,
**dadurch gekennzeichnet, dass** das Verbindungskabel (20) zum Anschluss des Feuchtigkeitssensors eine Anschlussklemme (21) nach einem der Ansprüche 1 bis 14 aufweist.

## Claims

1. Terminal for a moisture sensor which is to be placed on a patient's skin in order to monitor a vascular access, comprising a terminal tab on which electrical contact points are arranged, and having:
a first, lower leg (26) and a second, upper leg (27) for clamping the terminal tab, a rear end piece (34) of the lower leg (26) and a rear end piece (35) of the upper leg (37) being interconnected, and
terminal contacts (40) for contacting the electrical contact points of the terminal tab of the moisture sensor,
**characterised in that**
a front end piece (37) of the upper leg (27) comprises latching means (41) and a front end piece (28) of the lower leg (26) is configured both as a guide piece having a slot-shaped recess (30) for receiving the terminal tab of the moisture sensor, which recess extends over the width of the terminal and is laterally delimited by two stop elements (31a, 31b), the latching means (41) and the guide piece (28) being configured such that the latching means (41) is fixed in a snap-in manner in the slot-shaped recess (30) when the terminal is closed.

2. Terminal according to claim 1, **characterised in that** the front end piece (37) of the upper leg (27) has a contact surface (38) which faces the front end piece (28) of the lower leg (26) and on which the terminal contacts (40) are arranged.

3. Terminal according to either claim 1 or claim 2, **characterised in that** the front end piece (28) of the lower leg (26) has a support surface (39) for the terminal tab of the moisture sensor, which surface faces the front end piece (37) of the upper leg (27).

4. Terminal according to claim 3, **characterised in that** the support surface (39) for the terminal tab of the moisture sensor comprises a support element (46) made of a resilient material.

5. Terminal according to either claim 3 or claim 4, **characterised in that** the front end piece (37) of the upper leg (27) and the front end piece (28) of the lower leg (26) are configured such that the contact surface (38) and the support surface (39) are aligned in parallel with one another when the terminal is closed.

6. Terminal according to any of claims 1 to 5, **characterised in that** the guide piece (28) has a grip piece (29) located above the slot-shaped recess (30) and intended for opening the terminal.

7. Terminal according to any of claims 1 to 6, **characterised in that** the rear end pieces (34, 35) of the upper and lower legs (26, 27) are formed in one piece.

8. Terminal according to any of claims 1 to 7, **characterised in that** the rear end pieces (34, 35) of the upper and lower legs (26, 27) are configured as a connecting piece (42) which spring biases the upper and lower legs (26, 27) in the open position.

9. Terminal according to claim 8, **characterised in that** the upper and lower legs (26, 27) and the connecting piece (42) are configured such that the upper and lower legs are either not deformed or only minimally deformed when the terminal is opened and closed.

10. Terminal according to either claim 8 or claim 9, **characterised in that** the connecting piece (42) has a cross section which is substantially semicircular.

11. Terminal according to any of claims 1 to 10, **characterised in that** the upper and lower legs (26, 27) of the terminal are configured as flat parts made of plastics material.

12. Terminal according to any of claims 1 to 11, **characterised in that** the terminal has a socket (44) for connecting a plug of a connecting cable.

13. Arrangement comprising a moisture sensor (18) which is to be placed on a patient's skin and comprises a terminal tab (24) on which electrical contact points (23) are arranged, and a terminal (21) according to any of claims 1 to 12, **characterised in that** the width of the terminal tab (24) of the slot-shaped recess (30) of the guide piece (28) is dimensioned such that the terminal tab can be pushed into the guide piece so as to fit therein.

14. Arrangement according to claim 13, **characterised in that** the terminal tab (24) comprises a shoulder (25a, 25b) on each side, which shoulder rests against the stop elements (31a, 31b) of the guide piece (28) when the terminal tab (24) is pushed into the guide piece (28).

15. Device for monitoring a vascular access for an extracorporeal blood treatment device, in which a patient's blood is carried away from the patient via an arterial hose line (6) having an arterial cannula (5) and is supplied to the patient via a venous hose line (7) having a venous cannula (8), said device comprising:
a moisture sensor (18) which is to be placed on the patient's skin and comprises a terminal tab (24) on which electrical contact points (23) are arranged, and
an evaluation unit (19) which can be connected to the moisture sensor (18) via a connecting cable (20),
**characterised in that** the connecting cable (20) for connecting the moisture sensor comprises a terminal (21) according to any of claims 1 to 14.

## Revendications

1. Borne de raccord destinée à un capteur d'humidité à poser sur la peau d'un patient aux fins de la surveillance d'un accès vasculaire, qui présente une bride de raccord, au niveau de laquelle sont disposés des points de contact électriques, comprenant
une première branche inférieure (26) et une deuxième branche supérieure (27) servant à la fixation par serrage de la bride de raccord, une pièce d'extrémité arrière (34) de la branche inférieure (26) et une pièce d'extrémité arrière (35) de la branche supérieure (37) étant reliées entre elles, et
des contacts de raccord (40) servant à établir un contact avec les points de contact électriques de la bride de raccord du capteur d'humidité,
**caractérisée en ce que**
une pièce d'extrémité avant (37) de la branche supérieure (27) présente des moyens d'enclenchement (41), et **en ce qu'**une pièce d'extrémité avant (28) de la branche inférieure (26) est réalisée sous la forme d'une pièce de guidage pourvue d'un évidement (30) présentant une forme d'entaille, s'étendant sur la largeur de la borne de raccord, servant à recevoir la bride de raccord du capteur d'humidité, lequel évidement est délimité latéralement par deux éléments de butée (31a, 31b), les moyens d'enclenchement (41) et la pièce de guidage (28) étant réalisés de telle manière que les moyens d'enclenchement (41) sont fixés par enclenchement dans l'évidement (30) présentant une forme d'entaille lorsque la borne de raccord est fermée.

2. Borne de raccord selon la revendication 1, **caractérisée en ce que** la pièce d'extrémité avant (37) de la branche supérieure (27) présente une face de contact (38) tournée vers la pièce d'extrémité avant (28) de la branche inférieure (26), au niveau de laquelle sont disposés les contacts de raccord (40).

3. Borne de raccord selon la revendication 1 ou 2, **caractérisée en ce que** la pièce d'extrémité avant (28) de la branche inférieure (26) présente une surface d'appui (39), tournée vers la pièce d'extrémité avant (37) de la branche supérieure (27), destinée à la bride de raccord du capteur d'humidité.

4. Borne de raccord selon la revendication 3, **caractérisée en ce que** la face d'appui (39) destinée à la bride de raccord du capteur d'humidité présente un élément d'appui (46) en un matériau élastique.

5. Borne de raccord selon la revendication 3 ou 4, **caractérisée en ce que** la pièce d'extrémité avant (37) de la branche supérieure (27) et la pièce d'extrémité avant (28) de la branche inférieure (26) sont réalisées de telle manière que la face de contact (38) et la face d'appui (39) sont orientées de manière parallèle l'une par rapport à l'autre lorsque la borne de raccord est fermée.

6. Borne de raccord selon l'une quelconque des revendications 1 à 5, **caractérisée en ce que** la pièce de guidage (28) présente au-dessus de l'évidement (30) présentant une forme d'entaille une pièce de préhension (29) servant à ouvrir la borne de raccord.

7. Borne de raccord selon l'une quelconque des revendications 1 à 6, **caractérisée en ce que** les pièces d'extrémité arrière (34, 35) de la branche supérieure et de la branche inférieure (26, 27) sont d'un seul tenant.

8. Borne de raccord selon l'une quelconque des revendications 1 à 7, **caractérisée en ce que** les pièces d'extrémité arrière (34, 35) de la branche supérieure et de la branche inférieure (26, 27) sont réalisées sous la forme d'une pièce de liaison (42), qui précontraint par ressort la branche supérieure et la branche inférieure (26, 27) dans la position ouverte.

9. Borne de raccord selon la revendication 8, **caractérisée en ce que** les branches supérieures et inférieures (26, 27) et la pièce de liaison (42) sont réalisées de telle manière que, lors de l'ouverture et de la fermeture de la borne de raccord, la branche supérieure et la branche inférieure ne se déforment pas ou seulement de manière négligeable.

10. Borne de raccord selon la revendication 8 ou 9, **caractérisée en ce que** la pièce de liaison (42) présente une section transversale, qui présente essentiellement une forme semi-circulaire.

11. Borne de raccord selon l'une quelconque des revendications 1 à 10, **caractérisée en ce que** la branche supérieure et la branche inférieure (26, 27) de la borne de raccord sont réalisées sous la forme de parties plates en matière plastique.

12. Borne de raccord selon l'une quelconque des revendications 1 à 11, **caractérisée en ce que** la borne de raccord présente une douille (44) servant au raccord d'un connecteur d'un câble de liaison.

13. Ensemble comprenant un capteur d'humidité (18) à poser sur la peau d'un patient, qui présente une bride de raccord (24), au niveau de laquelle sont disposés des points de contact électriques, et comprenant une borne de raccord (21) selon l'une quelconque des revendications 1 à 12, **caractérisé en ce que** la valeur de la largeur de la bride de raccord (24) de l'évidement (30), présentant une forme d'entaille, de la pièce de guidage (28) est telle que la bride de raccord peut être insérée par glissement de manière adaptée dans la pièce de guidage.

14. Ensemble selon la revendication 13, **caractérisé en ce que** la bride de raccord (24) présente au niveau des deux côtés un épaulement (25a, 25b), qui repose au niveau des éléments de butée (31a, 31b) de la pièce de guidage (28) lorsque la bride de raccord (24) est insérée par glissement dans la pièce de guidage (28).

15. Dispositif servant à surveiller un accès vasculaire destiné à un dispositif de traitement extracorporel du sang, dans le cadre duquel du sang d'un patient est prélevé du patient par l'intermédiaire d'une conduite flexible artérielle (6), qui présente une canule artérielle (5), et est ramené au patient par l'intermédiaire d'une conduite flexible veineuse (7), qui présente une canule veineuse (8),
comprenant un capteur d'humidité (18) à poser sur la peau du patient, lequel présente une bride de raccordement (24), au niveau de laquelle sont disposés des points de contact (23) électriques, et
une unité d'analyse (19), qui peut être raccordée, par l'intermédiaire d'un câble de liaison (20), au capteur d'humidité (18),
**caractérisé en ce que** le câble de liaison (20) servant au raccordement du capteur d'humidité présente une borne de raccord (21) selon l'une quelconque des revendications 1 à 14.
